Europäisches Patentamt

European Patent Office

Office européen des brevets

(11) Publication number : **0 399 631 B1**

# EUROPEAN PATENT SPECIFICATION

(45) Date of publication of patent specification :
**16.02.94 Bulletin 94/07**

(51) Int. Cl.⁵ : **A61K 31/565, // (A61K31/565, 31:135)**

(21) Application number : **90250128.7**

(22) Date of filing : **17.05.90**

(54) Use of gestodene for the production of pharmaceutical agents.

(30) Priority : **17.05.89 DE 3916381**

(43) Date of publication of application :
**28.11.90 Bulletin 90/48**

(45) Publication of the grant of the patent :
**16.02.94 Bulletin 94/07**

(84) Designated Contracting States :
**AT BE CH DE DK ES FR GB GR IT LI LU NL SE**

(56) References cited :
**EP-A- 0 310 542**
**The Antiprogestin Steroid RU486 and Human Fertility Control, Etienne-Emile Beaulieu & Sheldon J. Segal Editors, Plenum Press, N.Y. & London, 1985, pp. 27-47**
**J. Steroid Biochem. 1989, vol. 33, no. 6, pp. 1055-1061 Coletta, A.A.**
**Eur. J. Cancer 1990, vol. 26, no. 5, pp. 608-610 K. Pollow et al.**

(56) References cited :
**Chemical Abstracts, Band 105, Nr. 23, 8. Dezember 1986, S. 75, Zusammenfassung Nr. 203373h, Columbus, Ohio, US; M.J. Iqbal e tal.**
**Chemical Abstracts, Band 106, Nr. 23, 8. Juni 1987, S. 98, Zusammenfassung Nr. 189321b, Columbus, Ohio, US;M.J. Iqbal et al.**
**Chemical Abstracts, Band 109, Nr. 11, 12. September 1988, S. 92, Zusammenfassung Nr. 86472j, Columbus, Ohio, US; M.J. Iqbal et al.**
**Unlisted Drugs, Band 38, Nr. 10, Oktober 1986, S. 194, Chatham, N.J., US**

(73) Proprietor : **SCHERING AKTIENGESELLSCHAFT**
**Müllerstrasse 170/178 Postfach 65 03 11**
**D-13303 Berlin (DE)**

(72) Inventor : **Henderson, David, Dr.**
**Jahnstrasse 17**
**D-1000 Berlin 28 (DE)**
Inventor : **Baum,Michael, Prof.**
**22 Red Post Hill**
**Dulwich, London SE21 (GB)**
Inventor : **Colletta, Anthony Adrian, Dr.**
**4 Talfourd Place**
**Peckham, London SE15 (GB)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 399 631 B1

## Description

This invention relates to a new medicinal use of steroids having a 15,16-double bond, especially gestodene (17α-ethynyl-17β-hydroxy-18-methyl-4,15-estradien-3-one), and specifically for the production of pharmaceutical agents for treatment of carcinomas whose growth can be inhibited by the protein TGFβ Tumor Growth Factor β, Sporn et al., 1986, Science 233 , pages 532-534), especially for the production of pharmaceutical agents for treatment of breast carcinoma.

It is already known that triphenylethylene derivatives such as, for example, tamoxifen [(Z)-2-(4-(1,2-diphenyl-1-butenyl)-phenoxy)-N,N-dimethyl-ethylamine] are substances with great affinity for the estrogen receptor and act as competitive estrogen antagonists. Therefore, they are used to combat estrogen-receptor positive breast carcinoma (Lit.: Patterson et al., 1981 Japanese Journal of Cancer Clinics, Suppl. (November), pages 157-183).

Further, it has been found that such compounds stimulate the synthesis of the TGFβ protein in breast carcinoma cells (lit.: Knabbe et al., 1987, Cell 48, pages 417-428). The influence of this protein on cell growth depends on the respective cell type. For example, the growth of fibroblasts is stimulated by TGFβ. But it has also been shown that TGFβ is of decisive importance for inhibiting the growth of breast carcinoma cells, and specifically also including cells that exhibit no steroid hormone receptors. Breast carcinomas are usually of polyclonal structure, i.e., they consist of a mixed population of cells that typically includes cells with and without receptors for steroid hormones (Lit.: King et al., 1985, Cancer Res. 45, pages 294-304; Horwitz et al., 1985, Recent Progress in Hormone Research 41, pages 249-316).

It is assumed that the favorable, clinically proven influence of tamoxifen on breast carcinoma cells reflects a direct antagonistic effect transmitted by the estrogen receptor in connection with an additional effect originating from the increased TGFβ synthesis.

Previously, besides tamoxifen and its chemically related compounds, no other agents have been known that exert a favorable (i.e., increasing) effect on TGFβ synthesis.

This invention involves the use of compounds that stimulate the synthesis of TGFβ, e.g., in carcinomas of the type described here, e.g., to a greater extent than tamoxifen or its chemically related compounds, and makes available agents which, like tamoxifen, can be used to treat tumors whose growth can be inhibited by TGFβ and/or which are estrogen-dependent.

It has been found that gestodene (17α-ethynyl-17β-hydroxy-18-methyl-4,15-estradien-3-one) surprisingly is able to influence the synthesis of TGFβ more positively than tamoxifen and that surprisingly other steroids with a 15,16-double bond show tumor inhibiting activity. Steroids having a 15,16-double bond, especially gestodene, are able significantly to inhibit the growth of estrogen receptor-positive carcinoma cells in vitro; this ability to inhibit the growth of carcinomas goes along with an increased synthesis of TGFβ. This unexpected effect is not shown by gestogens such as levonorgestrel (D-(-)-17α-ethynyl-17β-hydroxy-18-methyl-4-estren-3-one), very close in structure to gestodene.

The results of radioreceptor measurements to detect TGFβ of T47D breast carcinoma cells treated in vitro with ethanol (0.1%) or in each case with 10 nM of dexamethasone (9α-fluoro-16α-methyl-11β, 17,21-trihydroxy-1,4-pregnadiene-3,20-dione) or estradiol (as controls) and in each case with 500 nM of the gestagens 3-keto-desogestrel (17α-ethynyl-17β-hydroxy-18-methyl-11-methylene-4-estren-3-one), levonorgestrel, medroxyprogesterone acetate (17β-acetoxy-6α-methyl-4-pregnene-3,20-dione), R 5020 (17α,21-dimethyl-19-nor-4,9-pregnadiene-3,20-dione) and gestodene as well as, for comparison, tamoxifen or toremifen are given in Table 1. Treatment with the test substances named and the detection of TGFβ were performed as follows:

## 1. Treatment of the cells

T47D cells (Keydar et al., (1979) Eur. J. Cancer 15, 659-670) were kept in serum-free culture (Dulbecco's MEM without phenol red) with the addition of:

2 mmol/l of L-glutamine
MEM-vitamins (Sigma Chemical Co.)
5 micrograms/ml of insulin (bovine)
5 micrograms/ml of transferrin (human)
2 micrograms/ml of fibronectin (bovine)
10 ng/ml of EGF (epidermal growth factor) (bovine)
0.5 nmole/l of $CuSO_4$
1 nmole/l of sodium selenite

at 37°C. The cells were then treated in this culture with one of the above substances for 24 hours: the substances were dissolved in ethanol and added to the cultures, and the final concentration in all cultures was

2

adjusted to 0.1%. A culture that had been treated only with ethanol was used as the control. Afterward, the culture supernatents were collected, 50 micrograms/ml of bovine serum albumin (BSA) was added, it was lyophilized and dissolved in about 10 ml of phosphate-buffered physiological saline solution (PBS). These solutions were dialyzed at 4°C with 50 mM/l of ammonium acetate 6 times for 12 hours. The dialyzates were again lyophilized.

2. Competitive Radioreceptor measurement

A549 human lung cancer cells (obtainable from ATCC) were used as a source of the receptors needed for measurement. 18 to 24 hours before the test, in 24 well cluster dishes, there was cultured 1.8 to 2.0 x $10^5$ cells per well in Dulbecco's MEM with 5% fetal calf serum (conditions: incubator, 37°C, atmosphere 5% $CO_2$ in air, 100% humidity). Afterward, the cells were washed with 1 ml/well of buffer solution A (Dulbecco's MEM 25 mM of HEPES, pH 7.4, 0.1% BSA). The lyophilizates obtained under 1. after the dialysis were dissolved in 1-2 ml of buffer solution A and 0.2 ml of this solution or dilutions of it were poured on the washed cells. Then they were incubated at room temperature for 2 hours (5% $CO_2$ in air) and then the cells were washed twice with ice-cold buffer solution B (Hank's buffered saline with 0.1% BSA). Then 0.2 ml of a 50 pM-solution of [125]iodine-TGFβ (about 50,000 cpm/well) in buffer solution A was poured into each well. The specimens were again incubated for 2 hours at room temperature as described above and then washed twice with 1 ml of ice-cold buffer solution B. After separating the cells at 37°C with 0.75 ml/well of buffer solution C (1% triton X 100, 10% glycerin, 2 mM HEPES, pH = 7.4), the bound radioactivity was measured in a gamma counter. The determination of the TGFβ concentration contained in the individual specimens was performed by comparison with a calibration curve established with pure TGFβ, and the TGFβ concentrations determined this way were standardized to the number of T47D breast carcinoma cells used (see table 1).

Table 1

## Production of TGFβ by T47D breast carcinoma cells in vitro

| Test substance | ng TGFβ/$10^6$ cells · 24 hours |
|---|---|
| ethanol 0.1% | 0.2* |
| dexamethasone, 10 nM | 0.2 |
| estradiol, 10 nM | 0.2 |
| (gestagens, 500 nM) | |
| 3-ketodesogestrel | 0.2 |
| levonorgestrel | 0.2 |
| medroxyprogesterone acetate | 0.2 |
| R5020 | 0.2 |
| gestodene | 3.32 ± 0.71 ** |
| tamoxifen | 1.92 ± 0.08** |
| toremifen | 1.60 ± 0.1** |

\* Detection limit in the specimen 0.2 ng/$10^6$ cells

\*\* Average value ± standard deviation, n = 4

Cells were also treated two other ways in the following investigations. Method 1 is a method for screening T47D- and MCF-cells; method 2 has been used for screening the gestodene analogues mentioned (other steroids with 15,16-double bonding), using the T47D cell line alone.

Abbreviations used:

SF =        serum free

DCC-FCS =   Dextran charcoal treated fetal calf serum

GF/C =        glass fiber filters/C

Method 1: Cells were seeded ($10^5/25cm^2$ flask) in 5 ml of phenol-red free medium containing 5% DCC-FCS and allowed to grow for 4 days until 30-40% confluent. The monolayers were then washed twice with PBS and the cells incubated with 5 ml/flask of SF-medium containing 2mM glutamine, 0.2U/ml insulin, 10ng/ml EGF, 5µg/ml transferrin, 2µg/ml fibronectin, 1nM selenic acid and 0.5 nM $CuSO_4$. After 24 hours this was replaced with 5ml of fresh SF-medium containing the experimental drugs in quadruplicate, delivered as 1000X ethanol solutions (final ethanol concentration was 0.1%). 36 hours later the cells were labelled for DNA synthesis measurements.

Method 3: Cells were seeded as above in 5 ml of phenol-red free medium containing 5% DCC-FCS and allowed to attach overnight. This medium was removed and replaced with 5ml of SF-medium (as above) containing the drugs in quadruplicate and the cells grown in the presence of drug for a further 6 days with medium/drug changes at 48 and 96 hours. After 6 days the cells were labelled for DNA synthesis measurements.

For both methods above, the following technique was employed for cell labelling and DNA precipitation. For the last 2 hours of culture, $^3$H-thymidine was added to 1µCl/ml and the cells incubated at 37°C. The cell monolayers were washed twice with ice-cold PBS, harvested and pelleted. The cell pellets were resuspended in 1ml of water and sonicated (50W, 6 X 10s bursts on ice) until no intact cells were visible on phase contrast microscopy. 600µl of this homogenate was added to 600µl of ice-cold 10% TCA and the precipitate allowed to develop for 60 minutes at 4°C. The precipitate was collected under suction on GF/C filters and washed with 20ml of ice-cold 5% TCA followed by 5ml of ice-cold methanol. The filters were dried at 100°C for 10 minutes and scintillation counted. (Taken from: Steroid Hormones - A Practical Approach, Edited by B. Green and R. Leake. IRL Press Oxford, 1987, pp. 216-217).

Method 1:

T47D cells:

| | |
|---|---|
| Ethanol control | 100% |
| 10nM E2 | 127 ± 18% |
| 10nM Dex | 89 ± 14% |
| 11nM DHT | 97 ± 11% |
| 1µM 3-keto-Des | 104 ± 12% |
| 1µM MPA | 87 ± 14% |
| 1µM R5020 | 79 ± 9% |
| 1µM Tamoxifen | 58 ± 11% |
| 1µM Gestodene | 36 ± 7% (p<0.001 versus control) |

MCF-7 Cells:

| | |
|---|---|
| Ethanol control | 100% |
| 10nM E2 | 158 ± 27% |
| 10nM Dex | 120 ± 16% |
| 10nM DHT | 105 ± 19% |
| 1µM 3-keto-Des | 91 ± 11% |
| 1µM MPA | 80 ± 16% |
| 1µM R5020 | 84 ± 9% |
| 1µM Tamoxifen | 47 ± 6% |
| 1µM Gestodene | 44 ± 9% (p<0.001 versus control) |

Method 2: All experiments with T47D cells (n=16)

| | |
|---|---|
| Ethanol control | 100% |
| 1µM ZK 31305 | 13 ± 5% |
| 1µM ZK 48391 | 24 ± 10% |
| 1µM ZK 37655 | 9 ± 5% |
| 1µM ZK 39393 | 15 ± 4% |
| 1µM Gestodene | 16 ± 5% |

| ZK 31 305 | 17α-Chlorethinyl-17β-hydroxy-4,15-estradiene-3-one |
| ZK 48 391 | 17-Hydroxy-4,15-pregnadiene-3-one |
| ZK 37 655 | 17α-Chlorethinyl-17β-hydroxy-18-methyl-11-methylene-4,15-estradiene-3-one |
| ZK-39 393 | 17α-Ethinyl-11β-chlor-17β-hydroxy-18-methyl-4,15-estradiene-3-one |

Since, as already explained, breast carcinomas are inhibited in their growth not only by increased TGFβ synthesis in the carcinoma cells but are also favorably influenced by antiestrogens (e.g., competitive estrogen antagonists), in producing a pharmaceutical agent suitable for the treatment of breast carcinoma, preferably at least one antiestrogen is used additionally as an active ingredient aside from steroids having a 15,16-double bond, especially gestodene.

The weight ratio of both components can be varied within a broad range. Thus the same amounts of steroids having a 15,16-double bond, especially gestodene, and antiestrogen as well as an excess of one of the two components can be used. Steroids having a 15,16-double bond, especially gestodene, and antiestrogen are used together, separately, simultaneously and/or sequentially in a weight ratio of essentially 1:50 to 50:1, preferably 1:30 to 30:1 and especially 1:15 to 15:1. Preferably, steroids having a 15,16-double bond, especially gestodene, and antiestrogen can be administered combined in one dosage unit.

According to this invention, gestodene can be used in amounts of 0.5 to 100 mg daily; generally, 0.5 to 50 mg of gestodene daily will do. Amounts of other 15,16-ene-steroids will be analogous, e.g., biologically equivalent to the stated amounts of gestodene. These amounts apply to use of steroids having a 15,16-double bond, especially gestodene, alone or together with an antiestrogen. Typically, steroids having a 15,16-double bond, especially gestodene, can be used for purposes of this invention analogously to use of tamoxifen.

Suitable 15,16-ene-steroids are any which have the 15,16-ene feature, especially 15,16-pregnenes, preferably 4,15-pregnadienes, 15,16-estrenes, preferably, 4,15-estradienes, most especially such steroids having the structural features: 3-one, 17-hydroxy (preferably in the 17β-position), 17α-ethinyl or -chloroethinyl, 18-methyl and/or 11β-chloro, inter alia.

As antiestrogen, suitable are competitive estrogen antagonists, i.e., those compounds that are able to displace estrogens from the corresponding receptor and those compounds that prevent the biosynthesis of estrogens by aromatization of the corresponding nonaromatic precursors (aromatase inhibitor).

As competitive antiestrogens, all known competitive antiestrogens are suitable that fulfill the above-mentioned condition. They can be used in about the same amounts as the already marketed antiestrogens, i.e., suitable daily doses are about 5-100 mg for tamoxifen or biologically equivalent amounts of another antiestrogen. As nonsteroidal antiestrogens, the following may be mentioned:

Tamoxifen = (Z)-2-[p-(1,2-diphenyl-1-butenyl)-phenoxy]-N,N-dimethyl-ethylamine.

Toremifen = (Z)-2-[p-(1,2-diphenyl-1-w-chlorobutenyl)-phenoxy]-N,N-dimethylethylamine,

Nafoxidine = 1-2-[4-(6-methoxy-2-phenyl-3,4-dihydro-1-naphthyl)-phenoxy]-ethylpyrrolidine, hydrochloride

Mer 25 = 1-{p(2-diethylaminoethoxy)-phenyl}-2-(p-methoxyphenyl)-1-phenylethanol and compounds of the 1,1,2-triphenylbut-1-ene type, especially the 1,1-bis(3'-acetoxyphenyl)-2-phenyl-but-1-ene (J. Cancer Res. Clin. Oncol. (1986), 112, pages 119-124).

Further, suitable as steroidal antiestrogens are: 11α-methoxy-17α-ethynyl-1,3,5(10)-estratriene-3,17β-diol, 16β-ethylestradiol and 11-(3,17β-dihydroxy-1,3,5(10)-estratrien-7α-yl)-undecanoic acid-(N-butyl-N-methyl)-amide (EP-A 0138 504).

As aromatase inhibitors, all compounds are suitable that inhibit the formation of estrogens from their precursors, such as, for example, the 1-methyl-androsta-1,4-diene-3,17-dione described in DE-A-33 22 285, the testolactone (17α-oxa-D-homoandrosto-1,4-diene-3,17-dione) described in the Journal of Clinical Endocrinology and Metabolism, 49, 672 (1979), the compounds androsta-4,6-diene-3,17-dione, androsta-4,6-dien-17β-ol-3-one acetate, androsta-1,4,6-triene-3,17-dione, 4-androstene-19-chloro-3,17-dione, 4-androstene-3,6,17-trione, described in "Endocrinology" 1973, Vol. 92, No. 3, page 874, the 19-alkinylated steroids described in DE-A-31 24 780, the 10-(1,2-propadienyl) steroids described in DE-A-31 24 719, the 19-thio-androstane derivatives described in EP-A-100 566, the 4-androsten-4-ol-3,17-dione and its esters described in "Endocrinology" 1977, vol. 100, No. 6, page 1684 and US-A-4,235,093, the 1-methyl-15α-alkyl-androsta-1,4-diene-3,17-diones described in DE-A-35 39 244, the 10β-alkinyl-4,9-(11)-estradiene derivatives described in DE-A-36 44 358, and the 1,2β-methylene-6-methylene-4-androstene-3,17-dione described in EP-A-0250262.

As nonsteroidal aromatase inhibitors, for example, [4-(5,6,7,8-tetrahydroimidazo[1,5α]-pyridin-5-yl]benzonitrile monohydrochloride] can be mentioned (Cancer Res., 48, pages 834-838, 1988) or

Typical doses are 1-1,000 mg of 1-methyl-androsta-1,4-diene-3,17-dione daily or biologically equivalent doses of other aromatase inhibitors.

Steroids having a 15,16-double bond, especially gestodene, and antiestrogen-active compounds can be administered to mammals including humans, for example, locally, topically, subcutaneously, enterally or parenterally. For enteral administration, suitable are tablets, dragees, capsules, pills, suspensions or solutions that can be produced in the usual way with the additives and vehicles common in galenicals. For local or topical use, vaginal suppositories or transdermal systems such as skin plasters are suitable, for example.

**Claims**

1.  Use of steroids having a 15,16-double bond for the preparation of pharmaceuticals for treating a tumor whose growth can be inhibited by TGFβ.

2.  Use of steroids having a 15,16-double bond for the preparation of pharmaceuticals for treating breast carcinoma according to claim 1.

3.  Use according to claim 1, wherein the steroid is gestodene.

4.  Use according to claim 2, wherein the steroid is gestodene.

5.  Use according to claim 1, wherein the steroid is a pregnene or an estrene.

6.  Use according to claim 1, wherein the steroid is 3-one-4,15-pregnadiene or -estradiene.

7.  Use according to claim 1 further comprising the use of an antiestrogen for the preparation of the pharmaceuticals.

8.  Use according to claim 2, further comprising the use of an antiestrogen for the preparation of the pharmaceuticals.

9.  Use according to claim 3, further comprising the use of an antiestrogen for the preparation of the pharmaceuticals.

10. Use according to claim 4, further comprising the use of an antiestrogen for the preparation of the pharmaceuticals.

11. Use according to claim 9, wherein the antiestrogen is tamoxifen.

12. Use according to claim 10, wherein the antiestrogen is tamoxifen.

13. Use according to claim 3, wherein the gestodene is in a form adapted for the administration of 0.5 to 100 mg/day.

14. Use according to claim 14, wherein the ratio of gestodene to antiestrogen is 1:50 to 50:1.

15. Use according to claim 9, wherein the gestodene and antiestrogen are administered in the same dosage unit.

16. Use according to claim 7, wherein the antiestrogen is of the competitive estrogen antagonistic type or is an aromatase inhibitor.

17. A pharmaceutical composition useful for the treatment of a tumor whose growth can be inhibited by TGFβ comprising effective amounts of a steroid having a 15,16-double bond and an antiestrogen.

18. A pharmaceutical composition comprising a steroid having a 15,16-double bond and an antiestrogen.

19. A pharmaceutical composition of claim 17, further comprising a pharmaceutically acceptable carrier.

20. A pharmaceutical composition of claim 18, further comprising a pharmaceutically acceptable carrier.

21. A composition of claim 19, wherein the steroid is gestodene.

22. A composition of claim 20, wherein the steroid is gestodene.

**23.** A composition of claim 19, wherein the steroid is a pregnene or an estrene.

**24.** A composition of claim 20, wherein the steroid is a pregnene or an estrene.

**25.** A composition of claim 19, wherein the steroid is a 3-one-4,15-pregnadiene or -estradiene.

**26.** A composition of claim 20, wherein the steroid is a 3-one-4,15-pregnadiene or -estradiene.

**27.** A pharmaceutical composition of claim 20, comprising 0.5-50 mg of gestodene and 5-100 mg of tamoxifen or a bioequivalent amount of another antiestrogen.


**Patentansprüche**

**1.** Verwendung von Steroiden mit einer 15,16-Doppelbindung zur Herstellung von Arzneimitteln zur Behandlung eines Tumors, dessen Wachstum durch TGFβ inhibiert werden kann.

**2.** Verwendung von Steroiden mit einer 15,16-Doppelbindung zur Herstellung von Arzneimitteln zur Behandlung von Brustkarzinomen nach Anspruch 1.

**3.** Verwendung nach Anspruch 1, worin das Steroid Gestoden ist.

**4.** Verwendung nach Anspruch 2, worin das Steroid Gestoden ist.

**5.** Verwendung nach Anspruch 1, worin das Steroid ein Pregnen oder ein Estren ist.

**6.** Verwendung nach Anspruch 1, worin das Steroid ein 4,15-Pregnen-3-on oder ein -Estradien-3-on ist.

**7.** Verwendung nach Anspruch 1, die des weiteren die Verwendung eines Antiestrogens für die Herstellung des Arzneimittels umfaßt.

**8.** Verwendung nach Anspruch 2, die des weiteren die Verwendung eines Antiestrogens für die Herstellung des Arzneimittels umfaßt.

**9.** Verwendung nach Anspruch 3, die des weiteren die Verwendung eines Antiestrogens für die Herstellung des Arzneimittels umfaßt

**10.** Verwendung nach Anspruch 4, die des weiteren die Verwendung eines Antiestrogens für die Herstellung des Arzneimittels umfaßt.

**11.** Verwendung nach Anspruch 9, wobei das Antiestrogen Tamoxifen ist.

**12.** Verwendung nach Anspruch 10, wobei das Antiestrogen Tamoxifen ist.

**13.** Verwendung nach Anspruch 3, worin Gestoden in einer Form vorliegt, die zur Anwendung von 0,5 bis 100 mg/Tag hergerichtet ist.

**14.** Verwendung nach Anspruch 13, wobei das Verhältnis von Gestoden zum Antiestrogen 1:50 bis 50:1 beträgt.

**15.** Verwendung nach Anspruch 9, wobei Gestoden und das Antiestrogen in der selben Dosierungseinheit zur Anwendung kommen.

**16.** Verwendung nach Anspruch 7, wobei das Antiestrogen ein kompetitiver Estrogenantagonist oder ein Aromatasehemmer ist.

**17.** Pharmazeutische Zusammensetzung zur Behandlung eines Tumors dessen Wachstum durch die Behandlung mit TGFβ inhibiert werden kann, enthaltend wirksame Mengen eines Steroids mit einer 15,16-Doppelbindung und ein Antiestrogen.

**18.** Pharmazeutische Zusammensetzung enthaltend ein Steroid mit einer 15,16-Doppelbindung und ein An-

EP 0 399 631 B1

tiestrogen.

**19.** Pharmazeutische Zusammensetzung nach Anspruch 17, dadurch gekennzeichnet, daß sie außerdem einen pharmazeutisch verträglichen Trägerstoff enthält.

**20.** Pharmazeutische Zusammensetzung nach Anspruch 18, dadurch gekennzeichnet, daß es einen pharmazeutisch verträglichen Trägerstoff enthält.

**21.** Pharmazeutische Zusammensetzung nach Anspruch 19, worin das Steroid Gestoden ist.

**22.** Pharmazeutische Zusammensetzung nach Anspruch 20, worin das Steroid Gestoden ist.

**23.** Pharmazeutische Zusammensetzung nach Anspruch 19, worin das Steroid ein Pregnen oder ein Estren ist.

**24.** Pharmazeutische Zusammensetzung nach Anspruch 20, worin das Steroid ein Pregnen oder ein Estren ist.

**25.** Pharmazeutische Zusammensetzung nach Anspruch 19, worin das Steroid ein 4,15-Pregnadien-3-on oder -Estradien-3-on ist.

**26.** Pharmazeutische Zusammensetzung nach Anspruch 20, worin das Steroid ein 4,15-Pregnadien-3-on oder -Estradien-3-on ist.

**27.** Pharmazeutische Zusammensetzung nach Anspruch 20, enthaltend 0,5-50 mg Gestoden und 5-100 mg Tamoxifen oder eine bioequivalente Menge eines anderen Antiestrogens.


## Revendications

**1.** Emploi de stéroïdes ayant une double liaison 15,16 pour préparer des produits pharmaceutiques en vue du traitement de tumeurs dont le développement peut être inhibé par le TGFβ.

**2.** Emploi de stéroïdes ayant une double liaison 15,16 pour la préparation de produits pharmaceutiques en vue de traiter un carcinome du sein, selon la revendication 1.

**3.** L'emploi selon la revendication 1, pour lequel le stéroïde est du gestodène.

**4.** Emploi selon la revendication 2, pour lequel le stéroïde est du gestodène.

**5.** Emploi selon la revendication 1, pour lequel le stéroïde est un prégnène ou un estrène.

**6.** Emploi selon la revendication 1, pour lequel le stéroïde est le 3-one-4,15-prégnadiène ou -estradiène.

**7.** Emploi selon la revendication 1, comprenant en outre un anti-estrogène pour la préparation des produits pharmaceutiques.

**8.** Emploi selon la revendication 2, comprenant en outre un anti-estrogène pour la préparation des produits pharmaceutiques.

**9.** Emploi selon la revendication 3, comprenant en outre un anti-estrogène pour la préparation des produits pharmaceutiques.

**10.** Emploi selon la revendication 4, comprenant en outre un anti-estrogène pour la préparation des produits pharmaceutiques.

**11.** Emploi selon la revendication 9, pour lequel l'anti-estrogène est du tamoxifen.

**12.** Emploi selon la revendication 10, pour lequel l'anti-estrogène est du tamoxifen.

**13.** Emploi selon la revendication 3, pour lequel le gestodène est sous une forme adaptée à l'administration

8

quotidienne de 1,5 à 100 mg.

14. Emploi selon la revendication 7, pour lequel le rapport du gestodène à l'anti-estrogène est compris entre 1:50 et 50:1.

15. Emploi selon la revendication 9, pour lequel le gestodène et l'anti-estrogène sont administrés dans la même dose unitaire.

16. Emploi selon la revendication 7, dans lequel l'anti-estrogène est du genre antagoniste d'estrogènes concurrents ou bien un inhibiteur d'aromatases.

17. Composition pharmaceutique pour le traitement de tumeurs dont le développement peut être inhibé par le TGFβ, composition qui comprend une proportion appropriée d'un stéroïde à double liaison 15, 16, avec un antioestrogène.

18. Composition pharmaceutique comprenant un stéroïde à double liaison 15, 16, avec un antioestrogène.

19. Composition selon la revendication 17, comprenant aussi un véhicule à usage pharmaceutique.

20. Composition selon la revendication 18, comprenant aussi un véhicule à usages pharmaceutiques.

21. Composition selon la revendication 19 dont le stéroïde est du gestodène.

22. Composition selon la revendication 20 dont le stéroïde est du gestodène.

23. Composition selon la revendication 19 dont le stéroïde est un prégène ou un estrène.

24. Composition selon la revendication 20 dont le stéroïde est un prégène ou un estrène.

25. Composition selon la revendication 19 dont le stéroïde est un 3-one-4,15-prégnadiène ou -estradiène.

26. Composition selon la revendication 20 dont le stéroïde est un 3-one-4,15-prégnadiène ou - estradiène.

27. Composition selon la revendication 20 comprenant de 0,5 à 50 mg de gestodène avec de 5 à 100 mg de tamoxifen ou une quantité équivalente biologiquement d'un autre antioestrogène.